# DEMANDE DE BREVET EUROPEEN

(11) **EP 2 072 033 A2**
(43) Date de publication de la demande: **24.06.2009**
(21) Numéro de dépôt: 08164281.1
(22) Date de dépôt: 12.09.2008
(51) Int. Cl.: A61K 8/39, A61K 8/91, A61Q 5/02, A61Q 5/12

(54) **Composition cosmétique comprenant au moins un polymère cationique particulier et au moins un ester d'acide gras en C8-C24 et de sorbitan oxyéthyléné comprenant 2 à 10 motifs d'oxyéthylène, et procédé de traitement cosmétique mettant en oeuvre ladite composition**

(30) Priorité: 14.09.2007 FR 0757593
(71) Demandeur: L'Oreal, 75008 Paris (FR)
(72) Inventeur: Biganska, Olga, 92340 Bourg La Reine (FR); Mahe, Véronique, 78740 VAUX SUR SEINE (FR)
(74) Mandataire: Dossmann, Gérard

(57) **Abrégé**

La présente invention concerne une composition comprenant dans un milieu cosmétiquement acceptable :
- (i) un ou plusieurs polymères cationiques qui est obtenu par polymérisation d'un mélange de monomères comprenant un ou plusieurs monomères vinyliques substitués par un ou plusieurs groupes amino, un ou plusieurs monomères vinyliques non ioniques hydrophobes, et un ou plusieurs monomères vinyliques associatifs, et
- (ii) un ou plusieurs esters d'acide gras en C₈-C₂₄ et de sorbitan oxyéthyléné comprenant de 2 à 10 motifs d'oxyéthylène.

La présente invention concerne également un procédé de traitement cosmétique la mettant en oeuvre et son utilisation comme shampoing ou après-shampoing.

## Description

La présente invention concerne une composition cosmétique pour le traitement des matières kératiniques, de préférence humaines et plus particulièrement des cheveux, comprenant dans un milieu cosmétiquement acceptable, au moins un polymère épaississant cationique particulier et au moins un ester d'acide gras en C₈-C₂₄ et de sorbitan oxyéthyléné comprenant 2 à 10 motifs d'oxyéthylène. La présente invention concerne également un procédé de traitement cosmétique des cheveux et du cuir chevelu mettant en oeuvre ladite composition cosmétique.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Outre leurs éventuelles propriétés lavantes et leurs qualités cosmétiques, les utilisateurs sont sensibles au fait que les compositions capillaires, en particulier les shampooings et les après-shampooings, n'agressent pas la peau ni les yeux, cette qualité étant particulièrement appréciée dans les shampooings pour enfants. En effet, les shampooings les plus performants peuvent provoquer des picotements dans l'oeil lorsque le produit dilué coule dans la sphère oculaire, ce qui arrive fréquemment chez les enfants. Par ailleurs, bon nombre de ces shampooings provoquent, chez les personnes présentant une peau sensible, des réactions d'inconfort telles que des rougeurs, des démangeaisons, des picotements.

Ainsi, pour remédier à l'ensemble des inconvénients décrits ci-dessus, il est connu d'utiliser des esters d'acide gras en C₈-C₂₄ et de sorbitan oxyéthyléné comprenant de 2 à 10 motifs d'oxyéthylène en tant que tensioactifs non ioniques dans des compositions capillaires.

Toutefois, les compositions comprenant ces esters d'acides gras oxyéthylénés ne présentent pas en général des qualités d'usage satisfaisantes, notamment en termes de viscosité et/ou de répartition sur les cheveux. En effet, ces esters d'acides gras oxyéthylénés rendent le plus souvent les compositions trop épaisses entravant ainsi leur écoulement et/ou leur répartition sur les cheveux en raison du fait qu'il existe des interactions chimiques importantes entre ces esters d'acides gras oxyéthylénés et d'autres composés tels que des agents de conditionnement présents dans ces compositions.

De plus, ces compositions à base d'esters d'acides gras oxyéthylénés présentent une texture inhomogène et évoluent sensiblement au cours du temps dans des conditions normales de stockage en fonction de la température, notamment au niveau de leur viscosité et de leur aspect visuel. Ainsi, ces compositions présentent un aspect visuel trouble ainsi qu'une texture filante à 45°C dans des conditions normales de stockage.

Par ailleurs, dans le cas où ces compositions à base d'esters d'acides gras oxyéthylénés comprennent également des agents antipelliculaires ou des agents produisant un aspect ou un effet nacré, irisé, moiré ou métallisé tels que la cyclodextrine, ces compositions ne sont pas encore suffisamment stables.

Il existe donc un réel besoin de mettre au point des compositions cosmétiques contenant des esters d'acide gras en C₈-C₂₄ et de sorbitan oxyéthyléné comprenant de 2 à 10 motifs d'oxyéthylène ne présentant pas l'ensemble des inconvénients décrits ci-dessus, c'est-à-dire qui présentent notamment de bonnes propriétés d'usage, un aspect visuel satisfaisant et qui soient stables dans le temps tout en procurant des propriétés cosmétiques satisfaisantes aux matières kératiniques, en particulier aux cheveux et au cuir chevelu.

La demanderesse a découvert, de façon surprenante, qu'il était possible de formuler des compositions pour le traitement cosmétique des matières kératiniques, ayant les propriétés recherchées, en utilisant dans ces compositions au moins un ester d'acide gras en C₈-C₂₄ et de sorbitan oxyéthyléné comprenant de 2 à 10 motifs d'oxyéthylène et au moins un polymère épaississant cationique spécifique, défini ci-après.

En effet, il a été constaté que l'utilisation dudit polymère cationique dans les compositions de la présente invention permettait d'améliorer les propriétés d'usage, notamment en terme de viscosité, la stabilité ainsi que la texture des produits cosmétiques et en particulier des shampooings et après-shampoings à base d'esters d'acides gras en C₈-C₂₄ et de sorbitan oxyéthyléné comprenant de 2 à 10 motifs d'oxyéthylène tout en conférant aux matières kératiniques et notamment aux cheveux des propriétés cosmétiques satisfaisantes par exemple au niveau du démêlage, de la souplesse, de la malléabilité, du gonflant et en particulier au niveau de la brillance.

Les compositions ainsi obtenues présentent de bonnes propriétés d'usages c'est-à-dire que ces compositions peuvent s'écouler et se répartir plus facilement sur l'ensemble de la chevelure.

Par ailleurs, la stabilité au stockage des compositions selon l'invention est également améliorée aussi bien à température ambiante (20-25 °C) qu'à 45 °C, notamment au niveau de leur aspect visuel et de leur viscosité. Par « stables » au sens de la présente invention, on entend que l'aspect visuel ainsi que la viscosité de ces compositions n'évoluent pas sensiblement au cours du temps dans des conditions normales de stockage, par exemple pendant les 12, de préférence 24 et de manière encore préférée 30 mois à température ambiante qui suivent leur fabrication ou par exemple pendant les 2 mois à 45°C qui suivent leur fabrication.

De plus, les compositions selon la présente invention présentent une texture non filante et fondante qui leur permet d'être plus facilement absorbées à la surface de la chevelure ou de la peau. De telles compositions présentent également une texture homogène.

Par ailleurs, la texture améliorée permet au produit, une fois déposé, sur les cheveux de rester un certain temps sans s'écouler. Cette texture améliorée voire gélifiée, permet d'utiliser des quantités moindres de produits.

Enfin, lorsque les compositions présentent un agent nacrant tel que la cyclodextrine, les compositions sont stables au cours du temps et conservent leur aspect nacré.

L'invention a donc pour objet des compositions de traitement cosmétique des matières kératiniques, de préférence humaines, en particulier des fibres kératiniques, et plus particulièrement des cheveux, comprenant dans un milieu cosmétiquement acceptable, un ou plusieurs esters d'acide gras en C₈-C₂₄ et de sorbitan oxyéthyléné comprenant de 2 à 10 motifs d'oxyéthylène et un ou plusieurs polymères cationiques spécifiques tels que définis ci-après.

Elle a aussi pour objet un procédé de traitement cosmétique des matières kératiniques, de préférence humaines, en particulier des fibres kératiniques, et plus particulièrement des cheveux, mettant en oeuvre la composition selon l'invention.

Elle concerne également l'utilisation de la composition selon l'invention comme shampoing ou après-shampoing.

D'autres objets et caractéristiques, aspects et avantages de l'invention apparaîtront encore plus clairement à la lecture de la description et des exemples qui suivent.

La composition de traitement cosmétique des matières kératiniques, de préférence humaines, en particulier des fibres kératiniques, et plus particulièrement des cheveux, comprend dans un milieu cosmétiquement acceptable :
- (i) un ou plusieurs polymères cationiques obtenus par polymérisation d'un mélange de monomères comprenant un ou plusieurs monomères vinyliques substitués par un ou plusieurs groupes amino, un ou plusieurs monomères vinyliques non ioniques hydrophobes, et un ou plusieurs monomères vinyliques associatifs,
- (ii) un ou plusieurs esters d'acide gras en C₈-C₂₄ et de sorbitan oxyéthyléné comprenant de 2 à 10 motifs d'oxyéthylène.

Par « matières kératiniques », on comprend les cheveux, les cils, les sourcils, la peau, les ongles, les muqueuses ou le cuir chevelu et plus particulièrement les cheveux et par« fibres kératiniques », on comprend les cheveux, les cils, les sourcils.

L'une des caractéristiques essentielles de l'invention est la présence d'un polymère cationique (i) qui est obtenu par polymérisation d'un mélange de monomères comprenant un ou plusieurs monomères vinyliques substitués par un ou plusieurs groupes amino, un ou plusieurs monomères vinyliques non ioniques hydrophobes, et un ou plusieurs monomères vinyliques associatifs.

Les monomères présents au sein du polymère cationique (i) sont différents les uns des autres.

De préférence, les polymères cationiques (i) sont des polymères épaississants. Au sens de la présente invention, on entend par polymère épaississant, un polymère qui introduit à 1 % dans une solution aqueuse ou hydroalcoolique à 30 % d'éthanol, et à pH = 7, permet d'atteindre une viscosité d'au moins 100 cps à 25°C et à un taux de cisaillement de 1s⁻¹. Cette viscosité peut être mesurée à l'aide d'un viscosimètre cône/plan (Rhéomètre Haake R600 ou analogue).

De préférence, ces polymères augmentent par leur présence la viscosité des compositions dans lesquelles ils sont introduits d'au moins 50 cps à 25°C et à un taux de cisaillement de 1s⁻¹.

Le ou les polymères cationiques (i) utilisés dans la composition selon l'invention, et leur procédé de fabrication, sont notamment décrits dans la demande internationale WO 2004/024779.

Par monomère vinylique, on entend au sens de la présente invention un monomère comprenant au moins un groupe R₀CH=C(R₀)-, dans lequel chaque R₀ représente indépendamment H, un groupe alkyle en C₁-C₃₀, -COOH, -CO-OR₀', -O-CO-R₀', -CO-NHR₀', ou -CO-NR₀'R₀", R₀' et R₀" représentant un groupe alkyle en C₁-C₃₀.

Ainsi, par exemple, au sens de la présente invention, les (méth)acrylates et les (méth)acrylamides sont des monomères vinyliques.

Les monomères vinyliques substitués par un ou plusieurs groupes amino utilisables pour la préparation du polymère cationique (i) utilisé dans la composition selon l'invention sont des monomères à insaturation éthylénique, basiques et polymérisables. Les groupes amino peuvent dériver de groupes alkyle mono, di- ou polyaminés, ou bien de groupes hétéroaromatiques contenant un atome d'azote. Les groupes amino peuvent être des amines primaires, secondaires ou tertiaires. Ces monomères peuvent être utilisés sous la forme d'amine ou sous la forme de sel.

De préférence, le ou les monomères vinyliques substitués par un ou plusieurs groupes amino sont choisis parmi :
- les (méth)acrylates de mono(alkyl en C₁-C₄)amino(alkyle en C₁-C₈),
- les (méth)acrylates de di(alkyl en C₁-C₄)amino(alkyle en C₁-C₈), de préférence les (méth)acrylates de di(alkyl en C₁-C₄)alkylamino(alkyle en C₁-C₆),
- les mono(alkyl en C₁-C₄)amino(alkyl en C₁-C₈)(méth)acrylamides,
- les di(alkyl en C₁-C₄)amino(alkyl en C₁-C₈)(méth)acrylamides,
- les (méth)acrylamides à groupement hétérocyclique contenant un atome d'azote,
- les (méth)acrylates à groupement hétérocyclique contenant un atome d'azote,
- les hétérocycles azotés à groupement(s) vinyle(s),
- et leurs mélanges.

A titre de monomères vinyliques substitués par un ou plusieurs groupes amino préférés, on peut citer :
- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle ;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique contenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.

Lorsque les monomères sont sous forme de sels, il peut s'agir de sels minéraux, tels que les sels chlorhydrate, sulfate et phosphate ; ou bien de sels d'acides organiques, tels que les sels acétate, maléate et fumarate.

Des monomères vinyliques substitués par un ou plusieurs groupes amino particulièrement préférés sont :
- le (méth)acrylate de 3-(N,N-diméthylamino)propyle,
- le N'-(3-N,N-diméthylamino)propyl(méth)acrylamide,
- le (méth)acrylate de 2-(N,N-diméthylamino)éthyle,
- le (méth)acrylate de 2-(N,N-diéthylamino)éthyle,
- le (méth)acrylate de 2-(tert-butylamino)éthyle,
- le 2-(N,N-diméthylamino)propyl(méth)acrylamide, et
- l'acrylate de 2-(N,N-diméthylamino)néopentyle.

Le ou les monomères vinyliques substitués par un ou plusieurs groupes amino représentent généralement de 10 à 70 % en poids, de préférence de 20 à 60 % en poids, mieux de 30 à 40 % en poids, par rapport au poids total du mélange de monomères.

Par monomère hydrophobe, on entend au sens de la présente invention, un monomère qui présente une solubilité dans l'eau inférieure à 10g pour 100 mL d'eau à une température de 20°C.

Le ou les monomères vinyliques non ioniques hydrophobes utilisables pour la préparation du polymère cationique (i) utilisé dans la composition selon l'invention sont généralement choisis parmi les composés répondant à la formule (I) ou (II) :

(I) CH₂=C(X)Z,

(II) CH₂=CH-OC(O)R,

dans lesquelles formules (I) et (II) :
X représente H ou un groupe méthyle ;
Z est choisi parmi les groupes -C(O)OR¹, -C(O)NH₂, -C(O)NHR¹, -C(O)N(R¹)₂, -C6H5, -C₆H₄R¹, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-NH-CH₂CH₂-urée, -SiR₃, -C(O)O(CH₂)ₓSiR₃, -C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ;
chaque R¹ représente indépendamment un groupe alkyle en C₁-C₃₀, un groupe alkyle en C₂-C₃₀ hydroxylé, ou un groupe alkyle en C₁-C₃₀ halogéné.

On peut citer en particulier les (méth)acrylates d'alkyle en C₁-C₃₀; les (alkyl en C₁-C₃₀)(méth)acrylamides ; le styrène, les styrènes substitués et en particulier le vinyltoluène (ou 2-méthylstyrène), le butylstyrène, l'isopropylstyrène, le para-chlorostyrène ; les esters de vinyle et en particulier l'acétate de vinyle, le butyrate de vinyle, le caprolate de vinyle, le pivalate de vinyle et le néodécanoate de vinyle ; les nitriles insaturés et en particulier le (méth)acrylonitrile et l'acrylonitrile ; et les silanes insaturés et en particulier le triméthylvinylsilane, le diméthyléthylvinylsilane, l'allyldiméthylphénylsilane, l'allyltriméthylsilane, le 3-acrylamidopropyltriméthylsilane, le méthacrylate de 3-triméthylsilylpropyle.

De préférence, le ou les monomères vinyliques non ioniques hydrophobes sont choisis parmi les esters d'acide acrylique et d'alkyle en C₁-C₃₀, les esters d'acide méthacrylique et d'alkyle en C₁-C₃₀, et leurs mélanges, tels que l'acrylate d'éthyle, le méthacrylate de méthyle, le méthacrylate de 3,3,5-triméthylcyclohexyle, et leurs mélanges.

Le ou les monomères vinyliques non ioniques hydrophobes représentent généralement de 20 à 80% en poids, de préférence de 20 à 70% en poids, mieux de 50 à 65 % en poids, par rapport au poids total du mélange de monomères.

Le ou les monomères vinyliques associatifs utilisables pour la préparation du polymère cationique (i) utilisé dans la composition selon l'invention sont généralement choisis parmi des composés ayant une extrémité (i') à insaturation(s) éthylénique(s) pour la polymérisation par addition avec d'autres monomères du système, une portion centrale (ii') polyoxyalkylène pour conférer des propriétés hydrophiles sélectives aux polymères, et une extrémité (iii') hydrophobe pour conférer des propriétés hydrophobes sélectives aux polymères.

L'extrémité (i') à insaturation(s) éthylénique(s) du ou des monomères vinyliques associatifs est de préférence dérivée d'un acide ou d'un anhydride mono ou di-carboxylique à insaturation(s) α, β-éthylénique(s), de préférence un acide ou un anhydride mono ou di-carboxylique en C₃ ou C₄. De façon alternative, l'extrémité (i') du monomère associatif peut être dérivée d'un éther allylique ou d'un éther vinylique ; d'un monomère non ionique uréthane substitué par un groupe vinyle, tel que divulgué dans le brevet US redélivré n°33,156 ou dans le brevet US 5,294,692 ; ou d'un produit de réaction urée substituée par un groupe vinyle, tel que divulgué dans le brevet US 5,011,978.

La portion centrale (ii') du ou des monomères vinyliques associatifs est de préférence un segment polyoxyalkylène comprenant 5 à 250, de préférence encore 10 à 120, et mieux 15 à 60 motifs oxydes d'alkylène en C₂-C₇. Des portions centrales (ii') préférées sont les segments polyoxyéthylène, polyoxypropylène, et polyoxybutylène comprenant 5 à 150, de préférence 10 à 100, et mieux 15 à 60 motifs oxydes d'éthylène, de propylène ou de butylène, et des séquences aléatoires ou non aléatoires de motifs oxydes d'éthylène, oxydes de propylène ou oxydes de butylène. De préférence, les portions centrales sont des segments polyoxyéthylène.

L'extrémité (iii') hydrophobe du ou des monomères vinyliques associatifs est de préférence un fragment hydrocarboné choisi parmi un groupe alkyle linéaire en C₈-C₄₀, un groupe alkyle en C₂-C₄₀ substitué par un groupe aryle, un groupe phényle substitué par un groupe alkyle en C₂-C₄₀, un groupe alkyle ramifié en C₈-C₄₀, un groupe alicyclique en C₈-C₄₀, et un ester complexe en C₈-C₈₀.

Par ester complexe, on entend au sens de la présente invention, tout ester différent d'un ester simple.

Par ester simple, on entend au sens de la présente invention, tout ester d'alcool aliphatique saturé en C₁-C₃₀ linéaire ou ramifié et non substitué.

Des exemples d'extrémités (iii') hydrophobes du ou des monomères vinyliques associatifs sont des groupes alkyles linéaires ou ramifiés ayant de 8 à 40 atomes de carbone, tels que les groupes capryle (C₈), isooctyle (C₈ ramifié), décyle (C₁₀), lauryle (C₁₂), myristyle (C₁₄), cétyle (C₁₆), cétéaryle (C₁₆-C₁₈), stéaryle (C₁₈), isostéaryle (C₁₈ ramifié), arachidyle (C₂₀), béhényle (C₂₂), lignocéryle (C₂₄), cérotyle (C₂₆), montanyle (C₂₈), mélyssile (C₃₀) et laccéryle (C₃₂).

Des exemples de groupes alkyles linéaires ou ramifiés, ayant 8 à 40 atomes de carbone et dérivés d'une source naturelle sont notamment les groupes alkyles dérivés de l'huile d'arachide hydrogénée, d'huile de soja, d'huile de canola (à prédominance C₁₈), et de l'huile de suif hydrogénée en C₁₆-C₁₈ ; et les terpénols hydrogénés en C₁₀-C₃₀, tels que le géraniol hydrogéné (en C₁₀ ramifié), le farnesol hydrogéné (C₁₅ ramifié) et le phytol hydrogéné (C₂₀ ramifié).

Des exemples de groupe phényle substitué par un groupe alkyle en C₂-C₄₀ sont les groupes octylphényle, nonylphényle, décylphényle, dodécylphényle, hexadécylphényle, octadécylphényle, isooctylphényle et sec-butylphényle.

Des groupes alicycliques en C₈-C₄₀ peuvent être, par exemple, des groupes dérivés de stérols d'origine animale, tels que le cholestérol, le lanostérol, le 7-déhydrocholestérol ; ou bien des groupes dérivés de stérols d'origine végétale, tels que le phytostérol, le stigmastérol et le campestérol ; ou bien les dérivés de stérols issus de microorganismes, tels que l'ergostérol et le mycrostérol. D'autres groupes alicycliques utilisables dans la présente invention sont par exemple les groupes cyclooctyle, cyclododécyle, adamantyle et décahydronaphtyle, et les groupes dérivés de composés alicycliques naturels tels que le pinène, le rétinol hydrogéné, le camphre et l'alcool isobornylique.

Les groupes alkyles en C₂-C₄₀ substitués par un groupe aryle peuvent être, par exemple, un groupe 2-phényléthyle, un groupe 2,4-diphénybutyle, un groupe 2,4,6-triphénylhexyle, un groupe 4-phénylbutyle, un groupe 2-méthyl-2-phényléthyle et un groupe 2,4,6-tri(1'-phényléthyl)phényle.

A titre d'esters complexes en C₈-C₈₀, de préférence en C₈-C₄₀, utilisables comme extrémité (iii'), on peut notamment citer l'huile de ricin hydrogénée (principalement le triglycéride de l'acide 12-hydroxystéarique) ; les 1,2-diacylglycérols tels que le 1,2-distéarylglycérol, le 1,2-dipalmitylglycérol, le 1,2-dimyristylglycérol ; les di-, tri- ou polyesters de sucres tel que le 3,4,6-tristéarylglucose, le 2,3-dilaurylfructose ; et les esters de sorbitane tels que ceux divulgués dans le brevet US 4,600,761.

Les monomères vinyliques associatifs utilisables selon l'invention peuvent être préparés par toute méthode connue dans l'art antérieur. On peut se référer par exemple aux brevets US 4,421,902, US 4,384,096, US 4,514,552, US 4,600,761, US 4,616,074, US 5,294,692, US 5,292,843 ; US 5,770,760 et US 5,412,142.

De préférence, le ou les monomères vinyliques associatifs utilisables selon l'invention sont choisis parmi les composés de formule (III) : 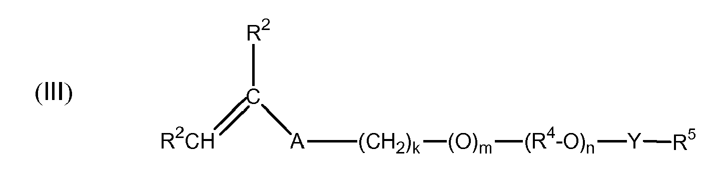 dans laquelle
chaque R² représente indépendamment H, un groupe méthyle, un groupe -C(O)OH, ou un groupe -C(O)OR³ ;
R³ représente un groupe alkyle en C₁-C₃₀ ;
A représente un groupe -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- ou -CH₂CH₂-NHC(O)- ;
Ar représente un groupe arylène ;
E représente H ou un groupe méthyle ;
z est égal à 0 ou 1 ;
k est un nombre entier allant de 0 à 30 ;
m est égal à 0 ou 1, à la condition que lorsque k = 0, m = 0, et lorsque k varie de 1 à 30, m est égal à 1 ;
(R⁴-O)ₙ représente un groupement polyoxyalkylène, qui est un homopolymère, un copolymère aléatoire, ou un copolymère à blocs, comportant des motifs oxyalkylène en C₂-C₄,
R⁴ représente -C₂H₄-, -C₃H₆-, -C₄H₈- ou leurs mélanges,
n est un entier variant de 5 à 250,
Y rerpésente -R⁴O-, -R⁴NH-, -C(O)-, -C(O)NH-, R⁴NHC(O)NH-, ou -C(O)NHC(O)- ;
R⁵ représente un groupe alkyle substitué ou non, choisi parmi les groupes alkyles linéaires en C₈-C₄₀, les groupes alkyles ramifiés en C₈-C₄₀, les groupes alicycliques en C₈-C₄₀, les groupes phényles substitués par un groupe alkyle en C₂-C₄₀, les groupes alkyles en C₂-C₄₀ substitués par un groupe aryle, et les esters complexes en C₈-C₈₀,
le groupe alkyle R⁵ comprenant éventuellement un ou plusieurs substituants choisis parmi les groupes hydroxyle, alcoxyle et halogéno.

De préférence, le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 motifs oxydes d'éthylène.

Plus particulièrement, le ou les monomères vinyliques associatifs sont choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, où la portion polyéthoxylée du monomère comprend de 10 à 80, de préférence de 15 à 60 et mieux de 20 à 40 motifs oxydes d'éthylène.

De préférence le ou les monomères vinyliques associatifs représentent de 0,001 à 25 % en poids, de préférence de 0,01 à 15 % en poids et mieux de 0,1 à 10 % en poids du mélange de monomères.

De préférence, le mélange de monomères pour l'obtention de polymères cationiques (i) contient en outre un ou plusieurs monomères semi-hydrophobes.

Le ou les monomères tensioactifs vinyliques semi-hydrophobes peuvent modérer les propriétés associatives des polymères associatifs cationiques qui les contiennent, produisant ainsi des gels aqueux ayant une très bonne texture et de très bonnes propriétés rhéologiques.

Par monomère tensioactif vinylique semi-hydrophobe, on entend au sens de la présente invention, on entend une structure similaire à un monomère associatif, mais qui a une extrémité substantiellement non hydrophobe et ainsi ne confère pas de propriété associative aux polymères.

La propriété d'associativité d'un polymère est liée à la propriété dans un milieu donné des molécules dudit polymère de s'associer entre elles ou de s'associer à des molécules d'un coagent (en général un tensioactif) ce qui se traduit dans un certain domaine de concentration à un accroissement supplémentaire de la viscosité du milieu.

Le ou les monomères tensioactifs vinyliques semi-hydrophobes sont généralement des composés ayant deux parties :
(i") un groupe terminal insaturé pour permettre la polymérisation par addition avec les autres monomères du mélange de réaction, et
(ii") un groupe polyoxyalkylène pour atténuer les associations entre les groupes hydrophobes du polymère ou les groupes hydrophobes des autres matériaux éventuellement présents dans la composition contenant le polymère.

L'extrémité fournissant l'insaturation vinylique ou éthylénique pour la polymérisation par addition est de préférence dérivée d'un acide ou d'un anhydride mono ou di-carboxylique à insaturation α, β-éthylénique, de préférence un acide mono ou di-carboxylique en C₃-C₄, ou un anhydride de cet acide. De façon alternative, l'extrémité (i") peut dériver d'un éther allylique, d'un éther vinylique ou d'un uréthane insaturé non ionique.

L'extrémité (i") insaturée polymérisable peut aussi dériver d'un acide gras insaturé en C₈-C₃₀ contenant au moins un groupe fonctionnel carboxy libre. Ce groupe en C₈-C₃₀ fait partie de l'extrémité insaturée (i") et est différent des groupes hydrophobes pendant des monomères associatifs, qui sont séparés de l'extrémité insaturée du monomère associatif par un groupe espaceur hydrophile.

La portion (ii") polyoxyalkylène comprend un segment polyoxyalkylène à chaîne longue, qui est essentiellement similaire à la portion hydrophile des monomères associatifs. Des portions polyoxyalkylène (ii") préférées incluent les motifs en C₂-C₄ polyoxyéthylène, polyoxypropylène, et polyoxybutylène comprenant de 5 à 250, de préférence de 10 à 100 motifs oxyalkylène. Lorsque le monomère tensioactif vinylique semi-hydrophobe comprend plus d'un type de motif oxyalkylène, ces motifs peuvent être disposés en séquence aléatoire, non aléatoire, ou à bloc.

De préférence, le ou les monomères tensioactifs vinyliques semi-hydrophobes sont choisis parmi les composés de formules (IV) ou (V) :

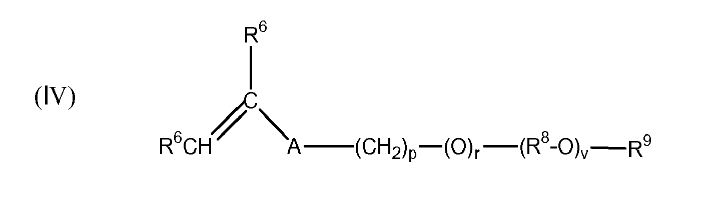

(V) D-A-(CH₂)ₚ-(O)ᵣ-(R⁸-O)ᵥ-R⁹

dans lesquelles formules (IV) et (V) :
chaque R⁶ représente indépendamment H, un groupe alkyle en C₁-C₃₀, -C(O)OH, ou -C(O)OR⁷ ;
R⁷ représente un groupe alkyle en C₁-C₃₀ ;
A représente un groupe -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- ou -CH₂CH₂NHC(O)- ;
Ar représente un groupe arylène ;
E représente H ou un groupe méthyle ;
z est égal à 0 ou 1 ;
p est un nombre entier allant de 0 à 30 ;
r est égal à 0 ou 1, à la condition que lorsque p est égal à 0, r est égal à 0, et lorsque p varie de 1 à 30, r est égal 1,
(R₈-O)ᵥ représente un groupement polyoxyalkylène qui est un homopolymère, un copolymère aléatoire, ou un copolymère à blocs avec des motifs oxyalkylène en C₂-C₄, où R⁸ représente -C₂H₄-, -C₃H₆-, -C₄H₈- ou leurs mélanges, et v est un nombre entier allant de 5 à 250;
R⁹ représente H ou un groupe alkyle en C₁-C₄ ;
D représente un groupe alcényle en C₈-C₃₀ ou un groupe alcényle en C₈-C₃₀ substitué par un groupe carboxy.

De manière particulièrement préférée, le mélange de monomères comprend un monomère tensioactif vinylique semi-hydrophobe ayant l'une des formules suivantes :

CH₂=CH-O(CH₂)ₐO(C₃H₆O)_{b}(C₂H₄O)_{c}H

ou

CH₂=CHCH₂O(C₃H₆O)_{d}(C₂H₄O)ₑH;

dans lesquelles :
a est égal à 2, 3, ou 4 ;
b est un nombre entier allant de 1 à 10 ;
c est un nombre entier allant de 5 à 50 ;
d est un nombre entier allant de 1 à 10 ; et
e est un nombre entier allant de 5 à 50.

Des monomères tensioactifs vinyliques semi-hydrophobes préférés sont, par exemple, les émulsifiants polymérisables commercialisés sous les références EMULSOGEN^{®} R109, R208, R307, RAL109, RAL208 et RAL307 par la société CLARIANT ; BX-AA-E5P5 commercialisé par la société BIMAX ; et le MAXEMUL^{®} 5010 et 5011 commercialisé par la société UNIQEMA. Les monomères particulièrement préférés sont l'EMULSOGEN^{®} R208, R307 et RAL 307.

Selon les fabricants :
l'EMULSOGEN^{®} R109 est un éther vinylique 1,4-butanediol éthoxylé/propoxylé aléatoire ayant la formule empirique :

   CH₂=CH-O(CH₂)₄O(C₃H₆O)₄(C₂H₄O)₁₀H,
l'EMULSOGEN^{®} R208 qui est un éther vinylique 1,4-butanediol éthoxylé/propoxylé aléatoire ayant la formule empirique :

   CH₂=CH-O(CH₂)₄O(C₃H₆O)₄(C₂H₄O)₂₀H;
l'EMULSOGEN^{®} R307 qui est un éther vinylique 1,4-butanediol éthoxylé/propoxylé aléatoire ayant la formule empirique :

   CH₂=CH-O(CH₂)₄O(C₃H₆O)₄(C₂H₄O)₃₀H;
l'EMULSOGEN^{®} RAL 109 qui est un éther allylique éthoxylé/propoxylé aléatoire ayant la formule empirique :

   CH₂=CHCH₂-O(C₃H₆O)₄(C₂H₄O)₁₀H,
l'EMULSOGEN^{®} RAL 208 qui est un éther allylique éthoxylé/propoxylé aléatoire ayant la formule empirique :

   CH₂=CHCH₂-O(C₃H₆O)₄(C₂H₄O)₂₀H;
l'EMULSOGEN^{®} RAL 307 qui est un éther allylique éthoxylé/propoxylé aléatoire ayant la formule empirique :

   CH₂=CHCH₂-O(C₃H₆O)₄(C₂H₄O)₃₀H;
le MAXEMUL^{®} 5010 qui est un alcényle en C₁₂-C₁₅ carboxylé hydrophobe, éthoxylé avec 24 unités d'oxyde éthylène,
le MAXEMUL^{®} 5011 qui est un alcényle en C₁₂-C₁₅ carboxylé hydrophobe, éthoxylé avec 34 unités d'oxyde éthylène ;
et le BX-AA-E5P5 qui est un éther allylique éthoxylé/propoxylé aléatoire ayant la formule empirique :

   CH₂=CHCH₂-O(C₃H₆O)₅(C₂H₄O)₅H.

La quantité du ou des monomères tensioactifs vinyliques semi-hydrophobes utilisés dans la préparation des polymères cationiques, de préférence épaississants, peut varier largement et dépend, entre autres, des propriétés rhéologiques finales désirées pour le polymère. Elle varie de 0 à 25 % en poids, mieux de 0,01 à 25% en poids, de préférence de 0,1 à 10 % en poids par rapport au poids total du mélange de monomères.

Le ou les polymères cationiques (i) utilisés dans la composition selon l'invention sont préparés à partir d'un mélange de monomères pouvant contenir un ou plusieurs monomères vinyliques non ioniques hydroxylés.

Ces monomères sont des monomères à insaturation éthylénique comprenant un ou plusieurs substituants hydroxyle.

A titre de monomères vinyliques non ioniques hydroxylés, on peut citer les (méth)acrylates d'hydroxyalkyle en C₁-C₆, de préférence les (méth)acrylates d'hydroxyalkyle en C₁-C₄, tel que le méthacrylate de 2-hydroxyéthyle (HEMA), l'acrylate de 2-hydroxyéthyle (2-HEA) et l'acrylate de 3-hydroxypropyle ; les (hydroxyalkyl en C₁-C₄)(méth)acrylamides, tels que le N-(2-hydroxyéthyl)méthacrylamide, le N-(2-hydroxyéthyl)acrylamide, le N-(3-hydroxypropyl)acrylamide et le N-(2,3-dihydroxypropyl)acrylamide ; et leurs mélanges. On peut encore citer l'alcool allylique, l'éther monoallylique de glycérol, le 3-méthyl-3-butèn-1-ol, les précurseurs de l'alcool vinylique et leurs équivalents, tel que l'acétate de vinyle.

Lorsqu'il(s) est (sont) présent(s), le ou les monomères vinyliques non ioniques hydroxylés représentent généralement jusqu'à 10 % en poids du poids total du mélange de monomères. Il(s) représente(nt) donc de 0 à 10 % en poids du poids total du mélange de monomères. De préférence, le ou les monomères vinyliques non ioniques hydroxylés représentent de 0,01 à 10% en poids, mieux de 1 à 8%, et encore de 1 à 5% en poids par rapport au poids total du mélange de monomères.

Le ou les polymères cationiques (i) utilisés dans la composition selon l'invention sont préparés à partir d'un mélange de monomères qui peut comprendre un ou plusieurs monomères réticulants permettant d'introduire des ramifications et de contrôler la masse moléculaire.

Des agents réticulants polyinsaturés utilisables sont bien connus dans l'état de la technique. Des composés mono-insaturés présentant un groupe réactif capable de réticuler un copolymère formé avant, pendant ou après la polymérisation peuvent aussi être utilisés. D'autres monomères réticulants utilisables peuvent être des monomères polyfonctionnels contenant des groupes réactifs multiples tels que des groupes époxydes, isocyanates et des groupes silanes hydrolysables. De nombreux composés polyinsaturés peuvent être utilisés pour générer un réseau tridimensionnel partiellement ou substantiellement réticulé.

Des exemples de monomères réticulants polyinsaturés utilisables sont, par exemple, les monomères aromatiques polyinsaturés, tel que le divinylbenzène, le divinylnaphtylène et le trivinylbenzène ; les monomères alicycliques polyinsaturés, tel que le 1,2,4-trivinylcyclohexane ; les esters difonctionnels de l'acide phtalique tel que le phthalate de diallyle ; les monomères aliphatiques polyinsaturés, tels que les diènes, les triènes et les tétraènes, notamment l'isoprène, le butadiène, le 1,5-hexadiène, le 1,5,9-décatriène, le 1,9-décadiène et le 1,5 heptadiène.

D'autres monomères réticulants polyinsaturés utilisables sont, par exemple, les éthers polyalcényliques, tels que le triallylpentaérythritol, le diallylpentaérythritol, le diallylsaccharose, l'octaallylsaccharose et l'éther diallylique de triméthylolpropane ; les esters polyinsaturés de polyalcools ou de polyacides, tels que le di(méth)acrylate de 1,6-hexanediol, le tri(méth)acrylate de tétraméthylène, l'acrylate d'allyle, l'itaconate de diallyle, le fumarate de diallyle, le maléate de diallyle, le tri(méth)acrylate de triméthylolpropane, le di(méth)acrylate de triméthylolpropane et le di(méth)acrylate de polyéthylène glycol ; les alkylène-bisacrylamides tels que le méthylène-bisacrylamide et le propylène-bisacrylamide ; les dérivés hydroxylés et carboxylés du méthylène-bisacrylamide, tels que le N,N'-bisméthylol-méthylène-bisacrylamide ; les di(méth)acrylates de polyéthylèneglycol, tels que le di(méth)acrylate d'éthylèneglycol, le di(méth)acrylate de diéthylèneglycol et le di(méth)acrylate de triéthylèneglycol ; les silanes polyinsaturés tels que le diméthyldivinylsilane, le méthyltrivinylsilane, l' allyldiméthylvinylsilane, le diallyldiméthylsilane et le tétravinylsilane ; les stannanes polyinsaturés tels que le tétraallylétain et le diallyldiméthylétain.

Des monomères réticulants monoinsaturés utilisables et portant un groupe réactif peuvent être les N-méthylolacrylamides ; les N-alcoxy(méth)acrylamides, où le groupe alcoxy comporte de 1 à 18 atomes de carbone ; et les silanes hydrolysables insaturés tels que triéthoxyvinylsilane, le tris-isopropoxyvinylsilane et le méthacrylate de 3-triéthoxysilylpropyle.

Des monomères réticulants polyfonctionnels utilisables et contenant plusieurs groupes réactifs peuvent être, par exemple, les silanes hydrolysables tels que l'éthyltriéthoxysilane et l'éthyltriméthoxysilane ; les silanes hydrolysables époxydés tels que le 2-(3,4-époxycyclohexyl)éthyltriéthoxysilane et le 3-glycidoxypropyltriméthyoxysilane ; les polyisocyanates tels que le 1,4-diisocyanatobutane, le 1,6-diisocyanatohexane, le 1,4-phénylènediisocyanate et le 4,4'-oxybis(phénylisocyanate) ; les époxydes insaturés tels que le méthacrylate de glycidyle et l'allylglycidyléther ; les polyépoxydes tels que le diglycidyléther, le 1,2,5,6-diépoxyhexane, et l'éthylèneglycoldiglycidyléther.

Des monomères réticulants polyinsaturés particulièrement utilisables sont les polyols éthoxylés, tels les diols, les triols et les bis-phénols, éthoxylés avec 2 à 100 moles d'oxyde d'éthylène par mole de groupe fonctionnel hydroxyle et terminés par un groupe insaturé polymérisable tel qu'un vinyléther, un allyléther, un ester acrylate ou un ester méthacrylate. De tels monomères réticulants peuvent être par exemple le diméthacrylate éthoxylé de biphénol A, le diméthacrylate éthoxylé de biphénol F, et le triméthacrylate éthoxylé de triméthylol propane.

D'autres monomères réticulants éthoxylés utilisables dans la présente invention sont, par exemple, les agents réticulants dérivés des polyols éthoxylés divulgués dans le brevet US 6 140 435.

Des exemples particulièrement préférés de monomères réticulants sont des esters acrylates et méthacrylates de polyols ayant au moins deux groupes ester acrylate ou méthacrylate, tel que le triacrylate de triméthylolpropane (TMPTA), le diméthacrylate de triméthylolpropane, le diméthacrylate de triéthylène glycol (TEGDMA), et le diméthacrylate de bisphénol A éthoxylé (30) (EOBDMA).

Lorsqu'il(s) est (sont) présent(s), le ou les monomères réticulants représentent de préférence au plus 5% en poids par rapport au poids du mélange de monomères. Selon un mode de réalisation préféré, les monomères réticulants sont présents à une teneur allant de 0,001 à 5% en poids, de préférence de 0,05 à 2% en poids, mieux de 0,1 à 1% en poids par rapport au poids total du mélange de monomères.

Le mélange de monomères peut contenir en outre un ou plusieurs agents de transfert de chaîne. Les agents de transferts de chaîne sont des composés bien connus de l'état de la technique.

On peut citer en particulier les composés thiolés, les composés disulfures, tels que les mercaptans en C₁-C₁₈, les acides mercaptocarboxyliques, les esters d'acides mercaptocarboxyliques, les thioesters, les (alkyl en C₁-C₁₈)disulfures, les aryldisulfures, les thiols polyfonctionnels ; les phosphites et hypophosphites ; les composés halogénoalcanes, tel que le tétrachlorure de carbone, le bromotrichlorométhane ; et les agents de transfert de chaîne insaturés, tel que l'alpha-méthylstyrène.

Les thiols polyfonctionnels sont, par exemple, les thiols trifonctionnels, tel que le triméthylolpropane-tris-(3-mercaptopropionate), les thiols tétrafonctionnels, tel que le pentaérythritol-tétra-(3-mercaptopropionate), le pentaérythritol-tétra-(thioglycolate) et le pentaérythritol-tétra(thiolactate) ; les thiols hexafonctionnels, tel que le pentaérythritol-hexa-(thioglyconate).

De façon alternative, le ou les agents de transfert de chaîne peuvent être des agents de transfert de chaîne catalytiques qui réduisent le poids moléculaire des polymères d'addition lors de la polymérisation par radicaux libres des monomères vinyliques. On peut citer par exemple les complexes de cobalt, notamment les chélates de cobalt (II). Les agents de transfert de chaîne catalytiques peuvent souvent être utilisés à des concentrations faibles par rapport aux agents de transfert de chaîne thiolés.

De façon particulièrement préférée, on peut citer à titre d'agent de transfert de chaîne l'octyl mercaptan, le n-dodécyle mercaptan, le t-dodécyle mercaptan, l'hexadécyle mercaptan, l'octadécyle mercaptan (ODM), l'isooctyl 3-mercaptopropionate (IMP), le butyl 3-mercaptopropionate, l'acide 3-mercaptopropionique, le butyl thioglycolate, l'isooctyl thioglycolate, le dodécyle thioglycolate.

Lorsqu'il(s) est (sont) présents, le ou les agents de transferts de chaîne sont ajoutés au mélange de monomères de préférence jusqu'à 10% en poids par rapport au poids total du mélange de monomères. De préférence, le ou les agents de transfert de chaîne représentent de 0,1% à 5 % en poids par rapport au poids total de monomères.

Le mélange de monomères permettant la préparation du polymère cationique (i) utilisé dans la composition selon l'invention peut comprendre un ou plusieurs agents de stabilisation polymériques pour l'obtention de dispersions ou d'émulsions stables. De préférence, les polymères stabilisants sont solubles dans l'eau. On peut citer par exemple les polymères synthétiques, tels que les alcools polyvinyliques, les acétates polyvinyliques partiellement hydrolysés, la polyvinylpyrrolidone, les polyacrylamides, les polyméthacrylamides, les polymères d'addition carboxylés, les polyalkyles vinyle éthers ; les polymères naturels hydrosolubles, tels que la gélatine, les peptines, les alginates, la caséine, l'amidon ; les polymères naturels modifiés, tels que la méthylcellulose, l'hydroxypropylcellulose, la carboxyméthylcellulose, les hydroxyéthylcelluloses allyliques.

Les agents de stabilisation polymériques sont utilisés en une quantité au plus égale à 2 % en poids par rapport au poids total du mélange de monomères, de préférence en une quantité comprise 0,0001 et 1 % en poids, mieux entre 0,01 et 0,5 % en poids par rapport au poids total du mélange de monomères.

Selon un mode de réalisation préféré, le mélange de monomères comprend, par rapport au poids total du mélange de monomères :
a) de 10 à 70 % en poids d'un ou plusieurs monomères vinyliques substitués par un ou plusieurs groupes amino,
b) de 20 à 80 % en poids d'un ou plusieurs monomères vinyliques non ioniques hydrophobes,
c) de 0,001 à 25 % en poids d'un ou plusieurs monomères vinyliques associatifs,
d) de 0 à 25 % en poids d'un ou plusieurs monomères tensioactifs vinyliques semi-hydrophobes,
e) de 0 à 10 % en poids d'un ou plusieurs monomères vinyliques non ioniques hydroxylés,
f) de 0 à 5% en poids d'un ou plusieurs monomères réticulants,
g) de 0 à 10 % en poids d'un ou plusieurs agents de transfert de chaîne, et
h) de 0 à 2 % en poids d'un ou plusieurs agents de stabilisation polymérique.

De façon encore plus préférée, le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants,
g) de 0,001 à 10 % en poids du ou des agents de transfert de chaîne, et
h) de 0 à 2 % en poids du ou des agents de stabilisation polymériques.

Selon un mode de réalisation particulièrement préféré, le mélange de monomères permettant la préparation du polymère cationique (i) utilisé dans la composition selon l'invention comprend, par rapport au poids total du mélange de monomères :
a) de 20 à 50 % en poids d'un ou plusieurs monomères vinyliques substitués par un ou plusieurs groupes amino choisi parmi :
   - le (méth)acrylate de 3-(N,N-diméthylamino)propyle,
   - le N'-(3-N,N-diméthylamino)propyl(méth)acrylamide,
   - le (méth)acrylate de 2-(N,N-diméthylamino)éthyle,
   - le (méth)acrylate de 2-(N,N-diéthylamino)éthyle,
   - le (méth)acrylate de 2-(tert-butylamino)éthyle,
   - le 2-(N,N-diméthylamino)propyl (méth)acrylamide, et
   - l'acrylate de 2-(N,N-diméthylamino)néopentyle,
b) de 50 à 65 % en poids d'un ou plusieurs monomères vinyliques non ioniques hydrophobes choisis parmi les esters d'acide acrylique et d'alkyle en C₁-C₃₀, les esters de l'acide méthacrylique et d'alkyle en C₁-C₃₀, et leurs mélanges,
c) de 0,1 à 10 % en poids d'un ou plusieurs monomères vinyliques associatifs choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges,
d) de 0,1 à 10 % en poids d'un ou plusieurs monomères tensioactifs vinyliques semi-hydrophobes ayant l'une des formules suivantes :

   CH₂=CH-O(CH₂)ₐO(C₃H₆O)_{b}(C₂H₄O)_{c}H

   ou

   CH₂=CHCH₂O(C₃H₆O)_{d}(C₂H₄O)ₑH

   dans lesquelles :
   a est égal à 2, 3, ou 4 ;
   b est un nombre entier allant de 1 à 10 ;
   c est un nombre entier allant de 5 à 50 ;
   d est un nombre entier allant de 1 à 10 ; et
   e est un nombre entier allant de 5 à 50,
e) jusqu'à 10 % en poids d'un ou plusieurs monomères vinyliques non ioniques hydroxylés,
f) jusqu'à 5% en poids d'un ou plusieurs monomères réticulants,
g) jusqu'à 10 % en poids d'un ou plusieurs agents de transfert de chaîne, et
h) jusqu'à 2 % en poids d'un ou plusieurs agents de stabilisation polymérique.

Les polymères cationiques (i) encore plus préférés selon l'invention sont des polymères issus de la polymérisation du mélange de monomères suivants :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

Parmi les polymères cationiques (i) utilisés dans la composition selon l'invention, on peut notamment citer le composé commercialisé par la société NOVEON sous la dénomination AQUA CC et qui correspond à la dénomination INCI POLYACRYLATE-1 CROSSPOLYMER.

Le POLYACRYLATE-1 CROSSPOLYMER est le produit de la polymérisation d'un mélange de monomères comprenant :
- un méthacrylate de di(alkyl en C₁-C₄) amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé (20-25 moles de motif oxyde d'éthylène),
- un allyl éther de polyéthylèneglycol/polypropylèneglycol 30/5,
- un méthacrylate d'hydroxy(alkyle en C₂-C₆), et
- un diméthacrylate d'éthylèneglycol.

Le ou les polymères cationiques (i) utilisés dans les compositions selon l'invention représentent généralement de 0,01 à 10% en poids, de préférence de 0,05 à 5% en poids, et mieux de 0,1 à 1 % en poids par rapport au poids total de la composition.

Le ou les polymères cationiques (i) utilisés dans la composition selon l'invention peuvent être préparés par des techniques de polymérisation conventionnelles, telles que la polymérisation en émulsion, comme cela est bien connu dans le domaine des polymères. La polymérisation peut être effectuée par simple procédé discontinu, par procédé par addition contrôlée, ou bien la réaction peut être initiée dans un petit réacteur puis alors la masse des monomères peut être ajoutée de façon contrôlée dans le réacteur (procédé par ensemencement). Généralement, la polymérisation est menée à une température de réaction comprise entre 20 et 80°C, même si des températures supérieures ou inférieures peuvent être utilisées. Pour faciliter l'émulsification du mélange de monomères, la polymérisation par émulsion est effectuée en présence d'un tensioactif présent en une quantité variant de 1 à 10 % en poids, de préférence de 3 à 8% en poids, mieux de 5 à 7 % en poids, par rapport au poids total de l'émulsion. Le milieu de réaction de polymérisation par émulsion comprend également un ou plusieurs initiateurs radicalaires, de préférence en une quantité variante de 0,01 à 3 % en poids par rapport au poids total du mélange de monomères. La polymérisation peut être réalisée dans un milieu aqueux ou bien hydroalcoolique à un pH neutre ou faiblement alcalin. Dans une polymérisation typique, le mélange de monomères est ajouté sous agitation à une solution de tensioactifs émulsifiants, tel qu'un tensioactif non ionique, de préférence un éthoxylate d'alcool linéaire ou ramifié, ou un mélange de tensioactifs non ioniques et anioniques, tels que des sulfates d'alcool gras ou des alkyl sulfonates d'alcool gras, dans une quantité adaptée d'eau, dans un réacteur adapté, pour préparer l'émulsion de monomères. L'émulsion est désoxygénée au moyen de toute méthode connue, puis la réaction de polymérisation est initiée en ajoutant un catalyseur de polymérisation (amorceur) tel que le persulfate de sodium, ou tout autre catalyseur de polymérisation par addition adaptée, comme cela est bien connu dans le domaine des polymères. Le mélange réactionnel est agité jusqu'à ce que la polymérisation soit complète, généralement pendant une durée variant de 4 heures à 16 heures. L'émulsion de monomères peut être chauffée à une température comprise entre 20 et 80°C avant l'addition de l'amorceur, si cela est souhaité. La quantité de monomères n'ayant pas réagi peut être éliminée par addition d'une quantité supplémentaire de catalyseur. L'émulsion de polymère obtenue peut être retirée du réacteur et emballée pour être stockée ou utilisée. De façon optionnelle, le pH ou d'autres caractéristiques physiques ou chimiques de l'émulsion peuvent être ajustés avant de retirer l'émulsion du réacteur. Généralement, l'émulsion produite a une teneur totale en matières solides qui varie entre 10 et 40 % en poids. Généralement, la quantité totale de polymères dans l'émulsion obtenue varie entre 15 et 35% en poids, en général au plus 25 % en poids.

Des tensioactifs adaptés pour faciliter la polymérisation par émulsion peuvent être des tensioactifs non ioniques, anioniques, amphotères ou cationiques, ou leurs mélanges. Le plus souvent, des tensioactifs non ioniques ou anioniques, ou leurs mélanges sont utilisés.

Tous types de tensioactifs non ioniques, anioniques, amphotères ou cationiques classiquement utilisés dans les polymérisations en l'émulsion peuvent être utilisés.

Comme expliqué plus haut, la polymérisation peut être effectuée en présence d'un ou plusieurs amorceurs conduisant à la formation de radicaux libres. Ceux-ci peuvent être choisis parmi les composés persulfates inorganiques insolubles, tels que le persulfate d'ammonium, le persulfate de potassium, le persulfate de sodium ; les peroxydes tels que le peroxyde d'hydrogène, le peroxyde de benzoyle, le peroxyde d'acétyle, et le peroxyde de lauryle ; les hydroperoxydes organiques, tels que l'hydroperoxyde de cumen et l'hydroperoxyde de t-butyle ; les peracides organiques, tel que l'acide peracétique ; et les agents producteurs de radicaux libres solubles dans l'huile, tels que 2,2'-azobisisobutyronitrile, et leurs mélanges. Les peroxydes et les peracides peuvent être éventuellement activés avec des agents réducteurs, tel que le bisulfite de sodium ou l'acide ascorbique, les métaux de transition, l'hydrazine. Des initiateurs de radicaux libres particulièrement adaptés sont les initiateurs de polymérisation azoïque solubles dans l'eau, tels que les composés 2,2'-azobis(tert-alkyl) ayant un substituant hydrosolubilisant sur le groupe alkyle. Des catalyseurs de polymérisation azoïque préférés sont les initiateurs de radicaux libres VAZO^{®}, commercialisés par la société DuPont, tel que le VAZO^{®} 44 (2,2'-azobis(2-4,5-dihydroimidazolyl)propane), le VAZO^{®} 56 (2,2'-azobis(2-méthylpropio-namidine)dihydrochlorure), et le VAZO^{®} 68 (4,4'-azobis(acide 4-cyanovalérique)).

La composition selon l'invention comprend un ou plusieurs esters d'acide gras en C₈-C₂₄ et de sorbitan oxyéthyléné comprenant de 2 à 10 motifs d'oxyéthylène.

De manière préférée, la composition selon l'invention comprend un ou plusieurs esters d'acide gras en C₈-C₁₄ et de sorbitan oxyéthyléné comprenant de 2 à 10 motifs d'oxyéthylène.

Plus préférentiellement, la composition selon l'invention comprend un ou plusieurs esters d'acide gras en C₁₂ et de sorbitan oxyéthyléné comprenant de 2 à 10 motifs d'oxyéthylène, et de préférence 4 motifs d'oxyéthylène.

De manière encore plus préférée, la composition selon l'invention comprend du mono-laurate de sorbitan oxyéthylène à 4 OE. Ce composé est également connu sous le nom de polysorbate 21. Il est, entre autres, commercialisé sous la dénomination TWEEN 21 par la société UNIQEMA.

La composition selon l'invention comprend préférentiellement au moins 0,5 % en poids d'ester d'acide gras en C₈ à C₂₄ et de sorbitan oxyéthyléné comprenant de 2 à 10 unités oxyéthylène, par rapport au poids total de la composition. De préférence, elle comprend de 0,5 à 10 % en poids d'ester d'acide gras en C₈ à C₂₄ et de sorbitan oxyéthyléné comprenant de 2 à 10 motifs d'oxyéthylène, plus préférentiellement de 0,5 à 9 % en poids, encore plus préférentiellement de 0,5 à 8 % en poids, par rapport au poids total de la composition.

Les compositions conformes à la présente invention peuvent également contenir un ou plusieurs agents nacrants.

Par le terme "agent de nacrage" ou "agent nacrant", on entend un agent produisant un aspect ou un effet nacré, irisé, moiré ou métallisé.

De préférence, l'agent nacrant est une cyclodextrine.

Par cyclodextrine, on entend au sens de la présente invention, une cyclodextrine non chimiquement modifiée ou un dérivé de cyclodextrine correspondant à une modification chimique d'une cyclodextrine.

Les cyclodextrines utilisables à titre d'agent nacrant dans les compositions selon la présente demande sont notamment des oligosaccharides de formule : 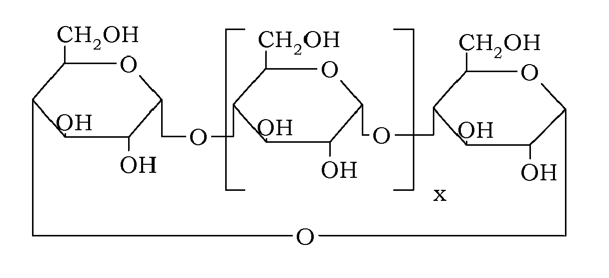 dans laquelle x peut être un nombre égal à 4 (ce qui correspond à l'α-cyclodextrine), à 5 (β-cyclodextrine) ou à 6 (γ-cyclodextrine).

De préférence la cyclodextrine est choisie parmi la β-cyclodextrine et la γ-cyclodextrine. De manière préférée il s'agit de la β-cyclodextrine.

On peut notamment utiliser une beta-cyclodextrine vendu par la société WACKER sous la dénomination CAVAMAX W7 PHARMA et une gamma cyclodextrine vendu par la société WACKER sous la dénomination CAVAMAX W8.

Les dérivés de cyclodextrines sont par exemple les méthyl cyclodextrines tels que la méthyl-beta-cyclodextrine commercialisée par la société WACKER sous la dénomination CAVASOL W7.

Selon l'invention, la ou les cyclodextrines représentent de 1 à 15 % en poids, de préférence de 1 % à 10 % en poids, et encore plus préférentiellement de 1,5 % à 5 % par rapport au poids total de la composition.

Les compositions selon l'invention peuvent se présenter sous la forme de shampooings, de compositions à appliquer avant ou après un shampooing, ces dernières se présentant sous la forme d'une lotion plus ou moins épaissie, d'un gel ou d'une émulsion.

Les compositions de l'invention peuvent ainsi comprendre un ou plusieurs tensioactifs choisis parmi les tensioactifs anioniques, cationiques, amphotères et non ioniques différents du monolaurate de sorbitane oxyéthyléné comprenant de 2 à 10 unités oxyéthylène.

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyllactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Lorsqu'ils sont présents, la quantité du ou des tensioactifs anioniques est de préférence comprise dans l'intervalle allant de 0,1 à 50 % en poids, mieux encore de 4 à 20% en poids par rapport au poids total de la composition.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels est de préférence comprise dans l'intervalle allant de 0,01 à 20 % en poids, mieux encore de 0,2 à 10 % en poids par rapport au poids total de la composition.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (VI) et (VII) :

Rₐ-CONHCH₂CH₂-N(R_{b})(R_{c})(CH₂COO⁻) (VI)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (VII)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques est de préférence comprise dans l'intervalle allant de 0,01 à 20 % en poids, mieux encore de 0,5 à 10% en poids par rapport au poids total de la composition.

Dans une variante préférée, la composition selon l'invention comprend au moins un tensioactif anionique et au moins un tensioactif amphotère ou zwittérionique.

La composition selon l'invention présente alors de préférence une teneur totale en tensioactifs anioniques, non-ioniques, amphotères et zwittérioniques comprise dans l'intervalle allant de 4 à 50 % en poids, mieux encore de 4 à 20 % en poids, par rapport au poids total de la composition.

Dans une seconde variante préférée, la composition cosmétique selon l'invention comprend en outre au moins un tensioactif cationique.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique ; les diesters quaternisés.

Lorsque les tensioactifs cationiques sont présents, leur quantité est de préférence comprise dans l'intervalle allant de 0,01 à 10 % en poids, mieux encore de 0,2 à 5 % en poids, et encore plus préférentiellement de 0,3 à 3 % en poids par rapport au poids total de la composition cosmétique.

Les compositions selon la présente invention peuvent comprendre en outre un ou plusieurs polymères cationiques différents des polymères cationiques (i).

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont une masse moléculaire moyenne en poids supérieure à 10⁵, de préférence supérieure à 10⁶ et mieux encore comprise entre 10⁶ et 10⁸.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) Les homopolymères ou copolymères dérivés d'esters ou d'amides acryliques ou méthacryliques et comportant au moins un des motifs de formules (VI), (VII), (VIII) ou (IX) suivantes: 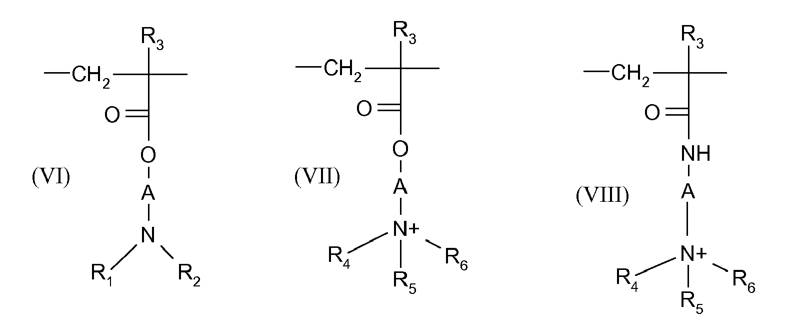 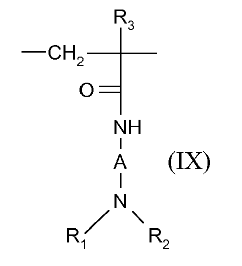 dans lesquelles:
   R₃, identiques ou différents, désignent un atome d'hydrogène ou un radical CH_{3;}
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un halogénure de diméthyle;
   - les copolymères d'acrylamide et de chlorure de méthacryloyloxyéthyltriméthylammonium décrits par exemple dans la demande de brevet EP-A-080976;
   - le copolymère d'acrylamide et de méthosulfate de méthacryloyloxyéthyltriméthylammonium;
   - les copolymères vinylpyrrolidone / acrylate ou méthacrylate de dialkylaminoalkyle quaternisés ou non. Ces polymères sont décrits en détail dans les brevets français 2.077.143 et 2.393.573;
   - les terpolymères méthacrylate de diméthyl amino éthyle/ vinylcaprolactame/ vinylpyrrolidone;
   - les copolymère vinylpyrrolidone / méthacrylamidopropyl diméthylamine;
   - les copolymères vinylpyrrolidone / méthacrylamide de diméthylaminopropyle quaternisés
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société CIBA.
(2) Les polysaccharides cationiques et en particulier ceux choisis parmi :
   (a) les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
   (b) les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylmidopropyl triméthylammonium, de diméthyl-diallylammonium.
   (c) les polygalactomannanes cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(4) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(7) Les cyclopolymères d'alkyl diallyl amine ou de dialkyl diallyl ammonium tels que les homopolymères ou copolymères comportant comme constituant principal de la chaîne des motifs répondant aux formules (X) ou (XI) : 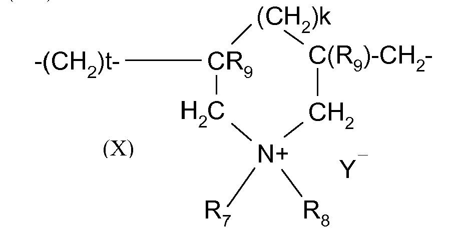 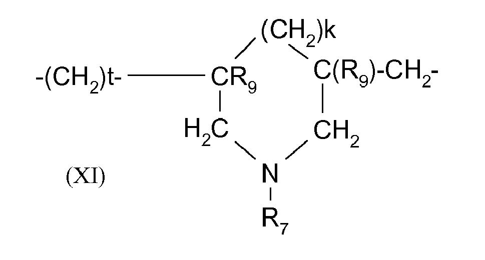 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
(8) Le polymère de diammonium quaternaire contenant des motifs récurrents répondant à la formule : 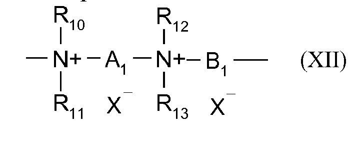 formule (XII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (XIII) suivante: 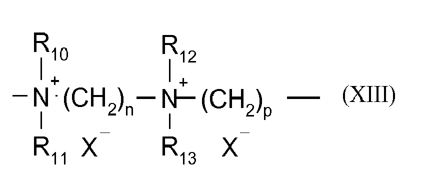 dans laquelle R₁₀, R₁₁, R₁₂ et R₁₃ identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
(9) Les polymères de poly(ammonium quaternaire) constitués de motifs récurrents de formule (XIV) : 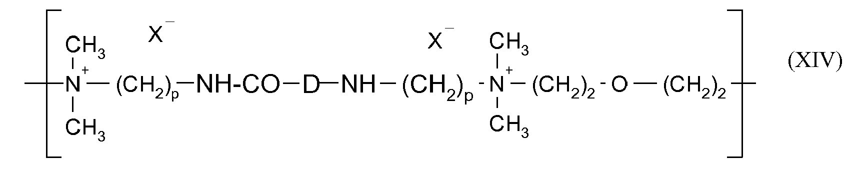 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
(10) Les polymères quaternaires de vinylpyrrolidone et de vinylimidazole.
(11) Les polyamines comme le produit référencé sous le nom de "POLYETHYLENEGLYCOL (15) TALLOW POLYAMINE" dans le dictionnaire CTFA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose comportant des groupements ammonium quaternaires tels que les produits vendus sous la dénomination "JR 400" par la Société AMERCHOL, les cyclopolymères cationiques, en particulier les homopolymères ou copolymères de chlorure de diméthyldiallylammonium, vendus sous les dénominations MERQUAT^{®} 100, MERQUAT^{®} 550 et MERQUAT^{®} S par la société NALCO, les gommes de guar modifiées par un sel de 2,3-époxypropyl-triméthylammonium, les polymères quaternaires de vinylpyrrolidone et de vinylimidazole et les polymères réticulés de sels méthacryloyloxyalkyl(C₁-C₄)trialkyl(C₁-C₄)ammonium réticulés.

Lorsque les polymères cationiques sont présents, ils sont de préférence présents en une quantité allant de 0,01 à 10 % en poids, mieux encore de 0,02 à 5 % en poids, et encore plus préférentiellement de 0,05 à 1 % en poids par rapport au poids total de la composition.

La composition selon l'invention peut comprendre en outre une ou plusieurs silicones.

Les silicones utilisables conformément à l'invention peuvent être volatiles ou non, solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les organopolysiloxanes sont définis plus en détail dans l'ouvrage de Walter NOLL "Chemistry and Technology of Silicones" (1968) Academic Press. Ils peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.

On peut également citer les cyclocopolymères du type diméthylsiloxane/méthylalkylsiloxane, tel que la "SILICONE VOLATILE FZ 3109" commercialisée par la société UNION CARBIDE, de structure chimique : avec D :

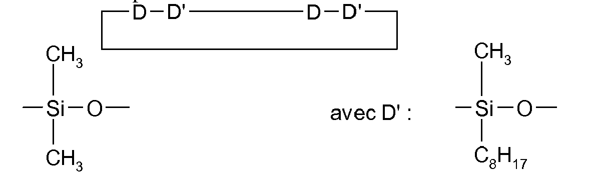

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer des polyalkylsiloxanes, des polyarylsiloxanes, des polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par Rhodia Chimie telles que par exemple l'huile 70 047 V 500 000 ;
- les huiles de la série MIRASIL commercialisées par la société Rhodia Chimie ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société Rhodia Chimie.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyl-diphénylsiloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de Rhodia Chimie,
- les huiles des séries RHODORSIL 70 633 et 763 de Rhodia Chimie ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes de polydiméthylsiloxane,
- les gommes polydiméthylsiloxanes/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane/diphénylsiloxane/ méthylvinylsiloxane.

Peuvent également être employés des mélanges de silicones tels que :
- les mélanges formés à partir d'un gomme de polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements ammonium quaternaires comme les produits commercialisés sous les dénominations ABILQUAT 3272 et ABILQUAT 3474 par la société GOLDSCHMIDT ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkylcarboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

Les silicones particulièrement préférées dans l'invention sont les polydiméthylsiloxanes tels que les polydiméthylsiloxanes à groupements terminaux triméthylsilyle, ou les polydiméthylsiloxanes à groupements terminaux hydroxydiméthylsilyle, et les silicones aminées.

Lorsque lesdites silicones sont présentes, elles sont contenues de préférence en une quantité allant de 0,05 à 20 % en poids, plus particulièrement de 0,1 à 10 % en poids, et mieux encore de 0,5 à 5 % en poids par rapport au poids total de la composition.

Par milieu cosmétiquement acceptable, on entend un milieu compatible avec les matières kératiniques, telles les cheveux et la peau.

Le milieu cosmétiquement acceptable est constitué d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention est généralement inférieur à 7, de préférence inférieur à 6, mieux compris entre 2 et 6, et encore plus préférentiellement entre 3 et 6.

La composition selon l'invention peut comprendre en outre un ou plusieurs additifs classiques bien connus dans la technique, tels que; des épaississants ou régulateurs de viscosité, naturels ou synthétiques ; des alcools gras en C₁₂-C₃₀ ; des céramides ; des esters gras huileux tels que le myristate d'isopropyle ou les triglycérides; des huiles minérales ou synthétiques telles que les α-oléfines ; des vitamines ou provitamines ; des agents de stabilisation du pH, des conservateurs ; et des colorants.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions conformes à l'invention peuvent être utilisées pour le lavage et le conditionnement des matières kératiniques, en particulier des cheveux, par exemple comme shampoings, ou encore pour le conditionnement des matières kératiniques, par exemple comme après-shampoings.

Un autre objet de l'invention est un procédé de traitement cosmétique des matières kératiniques, telles que les cheveux, qui consiste à appliquer une quantité efficace d'une composition telle que décrite ci-dessus, sur lesdites matières, à rincer après un éventuel temps de pose.

L'exemple suivant illustre la présente invention.

### EXEMPLE

On a préparé deux après-shampoings à partir des ingrédients indiqués dans le tableau ci-dessous.

| Compositions | A | B |
|---|---|---|
| Chlorure de sodium | 1 | 1 |
| Acide lactique | 0,343 | 0,286 |
| Acide salicylique (conservateur) | 0,15 | 0,2 |
| P-Hydroxybenzoate d'éthyle (conservateur) | 0,5 | 0,15 |
| Benzoate de sodium (conservateur) | 0,4 | 0,5 |
| P-Hydroxybenzoate de méthyle, sel de sodium (conservateur) | 0,5 | 0,4 |
| Parfum | 0,5 | 0,5 |
| Polyacrylate-1 Crosspolymer en émulsion à 20 % dans l'eau⁽¹⁾ | 3 | 3 |
| Hydroxyéthyl cellulose quaternisée par le chlorure de 2,3 époxypropyl triméthyl ammonium⁽²⁾ | 0,4 | - |
| Chlorure d'hydroxypropyl guar triméthyl ammonium ⁽³⁾ | - | 0,2 |
| Beta-cyclodextrine (cyclomaltoheptaose) ⁽⁴⁾ | 2 | 2 |
| Polydiméthylsiloxane (viscosité 500000 cSt ) ⁽⁵⁾ | 1,5 | - |
| Polydiméthylsiloxane ⁽⁶⁾ (viscosité 60000 cSt) | - | 2,7 |
| Monolaurate de sorbitan oxyéthyléné (4 OE) ⁽⁷⁾ | 6 | 0,2 |
| Cocoyl amidopropyl bétaïne en solution aqueuse (47% MA) ⁽⁸⁾ | 6,41 | 6,16 |
| Lauryl éther sulfate de sodium (2.2 OE) en solution aqueuse (70 %MA) ⁽⁹⁾ | 22,02 | 19,93 |
| Eau désionisée | Qsp 100g | Qsp 100g |

| | | |
|---|---|---|
| ⁽¹⁾ vendu sous la dénomination commerciale Carbopol Aqua CC par la société Noveon ⁽²⁾ vendu sous la dénomination commerciale JR400 par la société Amerchol ⁽³⁾ vendu sous la dénomination commerciale Jaguar C13S par la société Rhodia Chimie ⁽⁴) vendu sous la dénomination commerciale Cavamax W7 Pharma par lasociété WACKER ⁽⁵⁾ vendu sous la dénomination Dow Corning 200 Fluid 500000 par lasociété Dow Corning ⁽⁶⁾ vendu sous la dénomination Dow Corning 200 Fluid 60000 par la sociétéDow Corning ⁽⁷⁾ vendu sous la dénomination Tween 21 par la société Uniqema, ⁽⁸⁾ vendu sous la dénomination Tego Betaïne F50 par la sociétéGoldschmidt, ⁽⁹⁾ vendu sous la dénomination Texapon AOS 225UP par la société COGNIS | | |

Ces compositions présentent une bonne texture stable dans le temps. Elles sont peu agressives vis-à-vis du cuir chevelu, et appliquées sur les cheveux, permettent un bon lavage de ceux-ci avec en final l'apport de bonnes propriétés de conditionnement.

On a préparé une composition d'après shampooing à partir des ingrédients ci-après :

| Nom chimique | |
|---|---|
| Acide lactique | 0,343 |
| Chlorydrate de chlorhexidine (conservateur) | 0,02 |
| P-Hydroxybenzoate d'éthyle (conservateur) | 0,2 |
| Lanoline | 0,15 |
| Alcool cétylique | 2,5 |
| Parfum | 0,4 |
| Mélange myristate/palmitate/stéarate de myristyle/cétyle/stéaryle | 0,25 |
| Polyacrylate-1 Crosspolymer en émulsion à 20 % dans l'eau⁽¹⁾ | 2,5 |
| Hydroxyéthyl cellulose ⁽²⁾ | 0,2 |
| Polyméthyl lauryl / méthylsiloxane oxyéthylène (18 OE) et oxypropylène (18 OP) ⁽³⁾ | 0,15 |
| Polydiméthylsiloxane à groupements aminoéthyl iminopropyl à fonction méthoxy et/ou hydroxy et alpha-oméga silanols en émulsion aqueuse cationique ⁽⁴⁾ | 1,08 ' |
| Polyéthylène glycol (180 OE) ⁽⁵⁾ | 2 |
| Chlorure de cétyl triméthyl ammonium en solution aqueuse ⁽⁶⁾ | 1,8 |
| Mono-laurate de sorbitane oxyéthyléné (4 OE) ⁽⁷⁾ | 4 |
| Stéaryl amidopropyl diméthyl amine ⁽⁸⁾ | 0,75 |
| Eau désionisée | 84 |

| | |
|---|---|
| ⁽¹⁾ vendu sous la dénomination commerciale Carbopol Aqua CC par la société Noveon ⁽²⁾ vendu sous la dénomination commerciale Natrosol 250 HHR par la société Aqualon ⁽³⁾ vendu sous la dénomination commerciale Dow Corning 5200 par la société Dow Corning ⁽⁴⁾ vendu sous la dénomination commerciale Dow Corning 2-8299 par la société Dow Corning ⁽⁵⁾ vendu sous la dénomination Carbowax sentry PEG 8000 Granular NF/FCC par la société DOW CHEMICAL ⁽⁶⁾ vendu sous la dénomination Arquad 16-25 par la société AKZO NOBEL ⁽⁷⁾ vendu sous la dénomination Tween 21 par la société Uniqema ⁽⁸⁾ vendu sous la dénomination Mackine 301 par la société Mackintire | |

Cette composition présente une bonne texture stable dans le temps. Elle est peu agressive vis-à-vis du cuir chevelu et appliquée sur les cheveux en tant qu'après-shampooing elle confère aux cheveux de bonnes propriétés de démêlage.

## Revendications

1. Composition de traitement cosmétique des matières kératiniques, de préférence humaines, en particulier des fibres kératiniques, et plus particulièrement des cheveux, comprenant dans un milieu cosmétiquement acceptable :
- (i) un ou plusieurs polymères cationiques obtenus par polymérisation d'un mélange de monomères comprenant un ou plusieurs monomères vinyliques substitués par un ou plusieurs groupes amino, un ou plusieurs monomères vinyliques non ioniques hydrophobes et un ou plusieurs monomères vinyliques associatifs, et
- (ii) un ou plusieurs esters d'acide gras en C₈-C₂₄ et de sorbitan oxyéthyléné comprenant de 2 à 10 motifs d'oxyéthylène.

2. Composition selon la revendication 1, **caractérisée en ce que** le ou les monomères vinyliques substitués par un ou plusieurs groupes amino sont choisis parmi :
- les (méth)acrylates de mono(alkyl en C₁-C₄)amino(alkyle en C₁-C₈),
- les (méth)acrylates de di(alkyl en C₁-C₄)amino(alkyle en C₁-C₈), de préférence les (méth)acrylates de di(alkyl en C₁-C₄)alkylamino(alkyle en C₁-C₆),
- les mono(alkyl en C₁-C₄)amino(alkyl en C₁-C₈)(méth)acrylamides,
- les di(alkyl en C₁-C₄)amino(alkyl en C₁-C₈)(méth)acrylamides,
- les (méth)acrylamides à groupement hétérocyclique contenant un atome d'azote,
- les (méth)acrylates à groupement hétérocyclique contenant un atome d'azote,
- les hétérocycles azotés à groupement(s) vinyle(s),
- et leurs mélanges.

3. Composition selon l'une quelconque des revendications précédentes, **caractérisée en ce que** le ou les monomères vinyliques non ioniques hydrophobes répondent à la formule (I) ou (II) :
(I) CH₂=C(X)Z,
(II) CH₂=CH-OC(O)R;
dans lesquelles formules (I) et (II) :
X représente H ou un groupe méthyle ;
Z est choisi parmi les groupes -C(O)OR¹, -C(O)NH₂, -C(O)NHR¹, -C(O)N(R¹)₂, -C₆H₅, -C₆H₄R¹, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-NH-CH₂CH₂-urée, -SiR₃, -C(O)O(CH₂)ₓSiR₃, -C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ;
chaque R¹ représente indépendamment un groupe alkyle en C₁-C₃₀, un groupe alkyle en C₂-C₃₀ hydroxylé, ou un groupe alkyle en C₁-C₃₀ halogéné.

4. Composition selon l'une quelconque des revendications précédentes, **caractérisée en ce que** le ou les monomères vinyliques associatifs sont choisis parmi les composés de formule (III) : 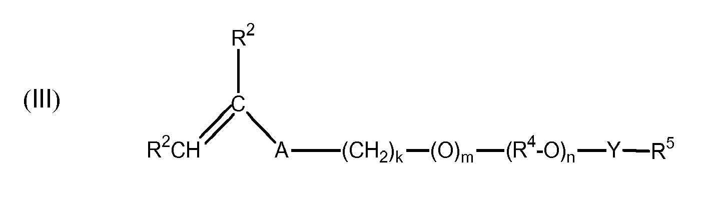 dans laquelle
chaque R² représente indépendamment H, un groupe méthyle, un groupe -C(O)OH, ou un groupe -C(O)OR³ ;
R³ représente un groupe alkyle en C₁-C₃₀ ;
A représente un groupe -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- ou -CH₂CH₂-NHC(O)- ;
Ar représente un groupe arylène ;
E représente H ou un groupe méthyle ;
z est égal à 0 ou 1 ;
k est un nombre entier allant de 0 à 30 ;
m est égal à 0 ou 1, à la condition que lorsque k = 0, m = 0, et lorsque k varie de 1 à 30, m est égal à 1 ;
(R⁴-O)ₙ représente un groupement polyoxyalkylène, qui est un homopolymère, un copolymère aléatoire, ou un copolymère à blocs, comportant des motifs oxyalkylène en C₂-C₄,
R⁴ représente -C₂H₄-, -C₃H₆-, -C₄H₈- ou leurs mélanges,
n est un entier variant de 5 à 250,
Y rerpésente -R⁴O-, -R⁴NH-, -C(O)-, -C(O)NH-, R⁴NHC(O)NH-, ou -C(O)NHC(O)- ;
R⁵ représente un groupe alkyle substitué ou non, choisi parmi les groupes alkyles linéaires en C₈-C₄₀, les groupes alkyles ramifiés en C₈-C₄₀, les groupes alicycliques en C₈-C₄₀, les groupes phényles substitués par un groupe alkyle en C₂-C₄₀, les groupes alkyles en C₂-C₄₀ substitués par un groupe aryle, et les esters complexes en C₈-C₈₀, le groupe alkyle R⁵ comprenant éventuellement un ou plusieurs substituants choisis parmi les groupes hydroxyle, alcoxyle et halogéno.

5. Composition selon l'une quelconque des revendications précédentes, **caractérisée en ce que** le mélange de monomères comprend un ou plusieurs monomères tensioactifs vinyliques semi-hydrophobes.

6. Composition selon la revendication 5, **caractérisée en ce que** le ou les monomères tensioactifs vinyliques semi-hydrophobes sont choisis parmi les composés de formules (IV) ou (V) : 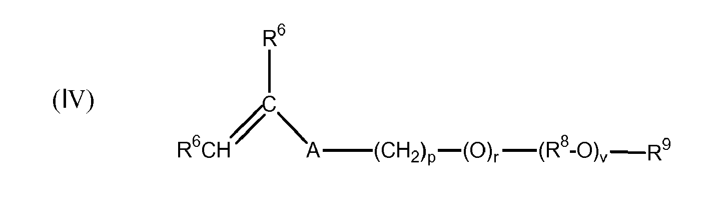
(V) D-A-(CH₂)ₚ-(O)ᵣ-(R⁸-O)ᵥ-R⁹
dans lesquelles formules (IV) et (V) :
chaque R⁶ représente indépendamment H, un groupe alkyle en C₁-C₃₀, -C(O)OH, ou -C(O)OR⁷;
R⁷ représente un groupe alkyle en C₁-C₃₀ ;
A représente un groupe -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- ou -CH₂CH₂NHC(O)- ;
Ar représente un groupe arylène ;
E représente H ou un groupe méthyle ;
z est égal à 0 ou 1 ;
p est un nombre entier allant de 0 à 30 ;
r est égal à 0 ou 1, à la condition que lorsque p est égal à 0, r est égal à 0, et lorsque p varie de 1 à 30, r est égal 1,
(R₈-O)ᵥ représente un groupement polyoxyalkylène qui est un homopolymère, un copolymère aléatoire, ou un copolymère à blocs avec des motifs oxyalkylène en C₂-C₄, où R⁸ représente -C₂H₄-, -C₃H₆-, -C₄H₈- ou leurs mélanges, et v est un nombre entier allant de 5 à 250 ;
R⁹ représente H ou un groupe alkyle en C₁-C₄ ;
D représente un groupe alcényle en C₈-C₃₀ ou un groupe alcényle en C₈-C₃₀ substitué par un groupe carboxy.

7. Composition selon l'une quelconque des revendications précédentes, **caractérisée en ce que** le ou les polymères cationiques (i) est ou sont obtenus par polymérisation du mélange de monomères comprenant, par rapport au poids total du mélange de monomères :
a) de 10 à 70 % en poids d'un ou plusieurs monomères vinyliques substitués par un ou plusieurs groupes amino,
b) de 20 à 80 % en poids d'un ou plusieurs monomères vinyliques non ioniques hydrophobes,
c) de 0,001 à 25 % en poids d'un ou plusieurs monomères vinyliques associatifs,
d) de 0 à 25 % en poids d'un ou plusieurs monomères tensioactifs vinyliques semi-hydrophobes,
e) de 0 à 10 % en poids d'un ou plusieurs monomères vinyliques non ioniques hydroxylés,
f) de 0 à 5% en poids d'un ou plusieurs monomères réticulants,
g) de 0 à 10 % en poids d'un ou plusieurs agents de transfert de chaîne, et
h) de 0 à 2 % en poids d'un ou plusieurs agents de stabilisation polymérique.

8. Composition selon l'une quelconque des revendications précédentes, **caractérisée en ce que** le ou les polymères cationiques (i) est ou sont obtenus par polymérisation du mélange de monomères comprenant, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants,
g) de 0,001 à 10 % en poids du ou des agents de transfert de chaîne, et
h) de 0 à 2 % en poids du ou des agents de stabilisation polymériques.

9. Composition selon l'une quelconque des revendications précédentes, **caractérisée en ce que** le polymère cationique (i) est obtenu par polymérisation du mélange de monomères suivants :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

10. Composition selon l'une quelconque des revendications précédentes, **caractérisée en ce que** l'ester d'acide gras en C₈ à C₂₄ et de sorbitan oxyéthyléné est un ester d'acide gras en C₁₂ et de sorbitan oxyéthyléné comprenant de 2 à 10 unités oxyéthylène.

11. Composition selon l'une quelconque des revendications précédentes, **caractérisée en ce que** l'ester d'acide gras en C₈ à C₂₄ et de sorbitan oxyéthyléné est le mono-laurate de sorbitan oxyéthyléné à 4 OE.

12. Composition selon l'une quelconque des revendications précédentes, **caractérisée en ce qu'**elle comprend en outre au moins une cyclodextrine choisie parmi l'α-cyclodextrine, la β-cyclodextrine, la γ-cyclodextrine chimiquement modifiées ou non.

13. Composition selon l'une quelconque des revendications précédentes, **caractérisée en ce qu'**elle comprend en outre un ou plusieurs tensioactifs anioniques et/ou non ioniques et/ou amphotères.

14. Composition la revendication 13, **caractérisée en ce que** le tensioactif anionique est choisi parmi les alkylsulfates, les alkyléthersulfates et leurs mélanges.

15. Composition selon la revendication 13, **caractérisée en ce que** le tensioactif amphotère ou zwittérionique est choisi parmi les (alkyl en C₈₋₂₀)-bétaïnes, les (alkyl en C₈₋₂₀)-amido(alkyl en C₆-₈)bétaïnes et leurs mélanges.

16. Procédé de traitement cosmétique des matières kératiniques, **caractérisé en ce que** l'on applique une quantité efficace d'une composition selon l'une quelconque des revendications précédentes, sur lesdites matières, à rincer après un éventuel temps de pose.

17. Utilisation d'une composition selon l'une quelconque des revendications 1 à 15, comme shampoing ou comme après-shampoing.
